(19) Europäisches Patentamt
European Patent Office
Office européen des brevets

(11) EP 0 689 839 A2

(12) **EUROPEAN PATENT APPLICATION**

(43) Date of publication:
**03.01.1996 Bulletin 1996/01**

(51) Int Cl.[6]: **A61K 31/71**

(21) Application number: **95304553.1**

(22) Date of filing: **28.06.1995**

(84) Designated Contracting States:
**AT BE CH DE DK ES FR GB GR IE IT LI LU NL PT SE**

(30) Priority: **30.06.1994 US 266406**

(71) Applicants:
- **ELI LILLY AND COMPANY**
  **Indianapolis, Indiana 46285 (US)**
- **ATHENA NEUROSCIENCES, INC.**
  **South San Francisco, CA 94080 (US)**

(72) Inventors:
- **Knops, Jeroen Elisabeth-Joseph**
  **San Francisco, California 94107 (US)**
- **Queener, Stephen Wyatt**
  **Indianapolis, Indiana 46208 (US)**
- **Sinha, Sukanto**
  **San Francisco, California 94127 (US)**

(74) Representative: **Tapping, Kenneth George et al**
**Windlesham, Surrey GU20 6PH (GB)**

(54) **Use of inhibitors of adenosine triphosphatase for the manufacture of a medicament for the treatment of disorders anociated with beta-amyloid peptide**

(57) This invention describes methods for treating conditions associated with an β-amyloid peptide, such as Alzheimer's disease or Down's Syndrome, in a mammal which comprises administering to the mammal in need thereof an effective amount of a derivative of a vacuolar adenotriphosphatase inhibitor or a pharmaceutically acceptable salt or prodrug thereof. This invention also describes methods for treating conditions associated with β-amyloid-associated toxicity in a mammal which comprises administering to the mammal an effective amount of a bafilomycin or concanamycin antibiotic or a pharmaceutically acceptable salt or prodrug thereof.

EP 0 689 839 A2

## Description

Alzheimer's disease is a degenerative disorder of the human brain. Clinically, it appears as a progressive dementia. Its histopathology is characterized by degeneration of neurons, gliosis, and the abnormal deposition of proteins in the brain. Proteinaceous deposits (called "amyloid") appear as neurofibrillary tangles, amyloid plaque cores, and amyloid of the congophilic angiopathy. [For a review, see, D.J. Selkoe, Neuron, 6:487-498 (1991)]

While there is no general agreement as to the chemical nature of neurofibrillary tangles, the major constituent of both the amyloid plaque cores and the amyloid of the congophilic angiopathy has been shown to be a 4500 Dalton protein originally termed β-protein or amyloid A4. Throughout this document this protein is referred to as β-amyloid peptide or protein.

β-Amyloid peptide is proteolytically derived from a transmembrane protein, the amyloid precursor protein. Different splice forms of the amyloid precursor protein are encoded by a widely expressed gene. see, e.g., K. Beyreuther and B. Müller-Hill, Annual Reviews in Biochemistry, 58:287-307 (1989). β-amyloid peptide consists, in its longest forms, of 42 or 43 amino acid residues. J. Kang, et al., Nature (London), 325:733-736 (1987). These peptides, however, vary as to their amino-termini. C. Hilbich, et al., Journal of Molecular Biology, 218:149-163 (1991).

Because senile plaques are invariably surrounded by dystrophic neurites, it was proposed early that β-amyloid peptide is involved in the loss of neuronal cells that occurs in Alzheimer's disease. B. Yankner and co-workers were the first to demonstrate that synthetic β-amyloid peptide could be neurotoxic in vitro and in vivo. B.A. Yankner, et al., Science, 245:417 (1989); See, also, N.W. Kowall, et al., Proceedings of the National Academy of Sciences, USA., 88:7247 (1991). Other research groups, however, were unable to consistently demonstrate direct toxicity with β-amyloid peptide. See, e.g., Neurobiology of Aging, 13:535 (K. Kosik and P. Coleman, eds. 1992). Even groups receiving β-amyloid peptide from a common source demonstrate conflicting results. P. May, et al., Neurobiology of Aging, 13:605-607 (1992).

In addition to Alzheimer's disease, Down's syndrome is also characterized by an accumulation of β-amyloid peptide. In patients suffering from Down's syndrome the β-amyloid peptide is the primary constituent of senile plaques and cerebrovascular deposits.

Because of the debilitating effects of Alzheimer's disease, Down's syndrome, and these other conditions associated with amyloidogenic peptides and proteins there continues to exist a need for effective treatments. This invention provides compounds efficacious in the treatment and prevention of these disorders.

This invention describes a method of treating or preventing a physiological disorder associated with β-amyloid peptide in a mammal which comprises administering to a mammal in need of said treatment an effective amount of an inhibitor of vacuolar adenosinetriphosphatase or a pharmaceutically acceptable salt or prodrug thereof.

More particularly this invention describes a method of treating or preventing a physiological disorder associated with β-amyloid peptide in a mammal which comprises administering to a mammal in need of said treatment an effective amount of a compound of Formula I

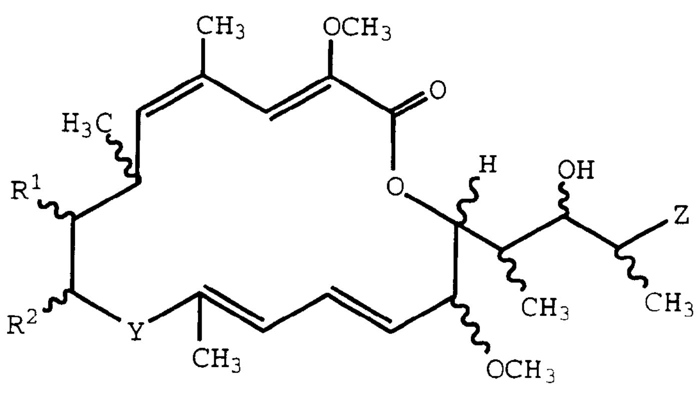

I

wherein:

Y is

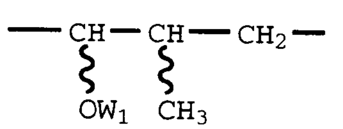

,

or $-CH_2-$;

Z is

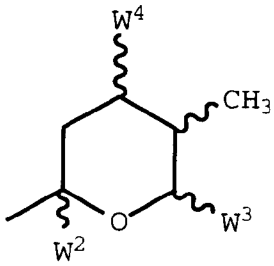

, or

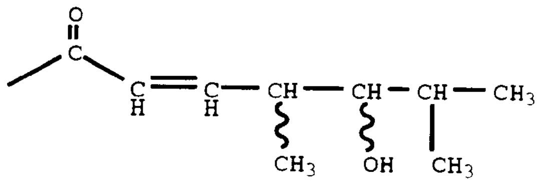

;

$R^1$ is hydroxy or $C_2$-$C_6$ alkanoyloxy;
$R^2$ is $C_1$-$C_6$ alkyl;
$W^1$ is hydrogen or $C_2$-$C_6$ alkanoyl;
$W^2$ is hydrogen, hydroxy, or $C_1$-$C_6$ alkoxy;
$W^3$ is $C_1$-$C_6$ alkyl, or $C_2$-$C_8$ alkenyl;
$W^4$ is hydroxy, $C_1$-$C_6$ alkoxy, $C_2$-$C_6$ alkanoyloxy,

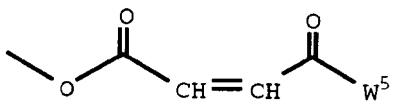

,

=O , or

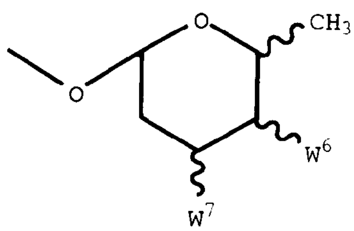

;

$W^5$ is hydroxy, $C_1$-$C_6$ alkoxy, amino,

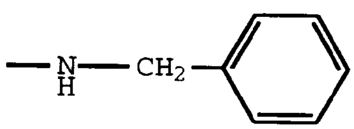

, or

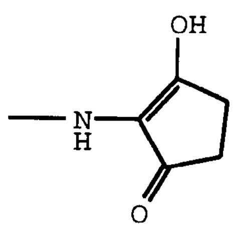

;

$W^6$ is hydroxy or $C_2$-$C_6$ alkanoyloxy;
$W^7$ is hydroxy or $C_2$-$C_6$ alkanoyloxy;
or a pharmaceutically acceptable salt, solvate, or prodrug thereof.

In another embodiment this invention describes a method of treating or preventing a physiological disorder associated with β-amyloid peptide in a mammal which comprises administering to a mammal in need of said treatment an effective amount of a compound of Formula II

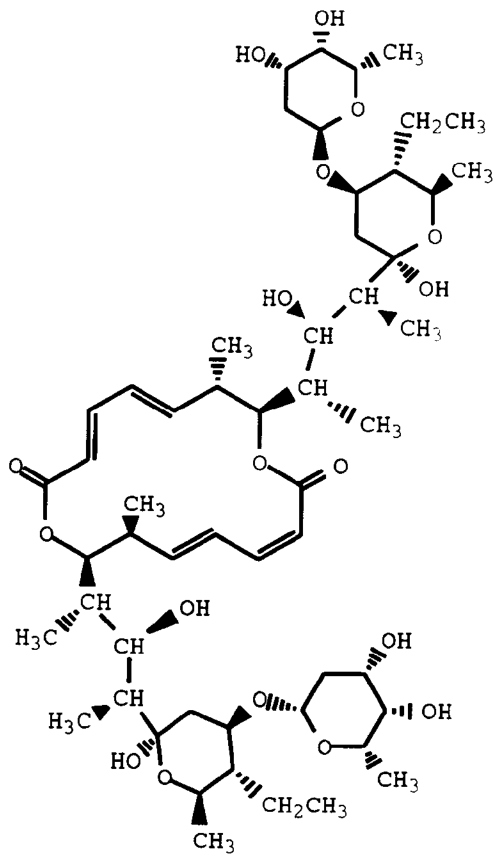

II

or a pharmaceutically acceptable salt, solvate, or prodrug thereof.

The terms and abbreviations used in the instant examples have their normal meanings unless otherwise designated. For example "°C" refers to degrees Celsius; "N" refers to normal or normality; "mmol" refers to millimole or millimoles; "g" refers to gram or grams; "ml" means milliliter or milliliters; "M" refers to molar or molarity; "FDMS" refers to field desorption mass spectrometry; "IR" refers to infrared spectroscopy; and "NMR" refers to nuclear magnetic resonance spectroscopy.

As used herein, the term "$C_1$-$C_6$ alkyl" refers to straight or branched, monovalent, saturated aliphatic chains of 1 to 6 carbon atoms and includes, but is not limited to, methyl, ethyl, propyl, isopropyl, butyl, isobutyl, t-butyl, pentyl, isopentyl, and hexyl. The term "$C_1$-$C_6$ alkyl" includes within its definition the term "$C_1$-$C_3$ alkyl".

The term "$C_2$-$C_8$ alkenyl" as used herein represents a straight or branched, monovalent, unsaturated aliphatic chain having from two to eight carbon atoms. Typical $C_2$-$C_6$ alkenyl groups include ethenyl (also known as vinyl), 1-methylethenyl, 1-methyl-l-propenyl, 1-butenyl, 1-hexenyl, 2-methyl-2-propenyl, 1-propenyl, 2-propenyl, 2-butenyl, 2-pentenyl, and the like.

"$C_1$-$C_6$ alkoxy" represents a straight or branched alkyl chain having from one to six carbon atoms attached to an oxygen atom. Typical $C_1$-$C_6$ alkoxy groups include methoxy, ethoxy, propoxy, isopropoxy, butoxy, t-butoxy, pentoxy and the like. The term "$C_1$-$C_6$ alkoxy" includes within its definition the term "$C_1$-$C_3$ alkoxy".

"$C_2$-$C_6$ alkanoyl" represents a straight or branched alkyl chain having from one to five carbon atoms attached to a carbonyl moiety. Typical $C_2$-$C_6$ alkanoyl groups include ethanoyl, propanoyl, isopropanoyl, butanoyl, *t*-butanoyl, pentanoyl, hexanoyl, 3-methylpentanoyl and the like.

The term "$C_2$-$C_6$ alkanoyloxy" refers to a $C_2$-$C_6$ alkanoyl radical, as defined supra, attached through an oxygen atom. An especially preferred moiety of this group is acetoxy, having the structure $-OC(O)CH_3$.

As used herein the term "inhibit" or "inhibiting" includes its generally accepted meaning which encompasses prohibiting, preventing, restraining, and slowing, stopping, or reversing progression, severity, or a resultant symptom. As such, the methods of this invention encompass both therapeutic and prophylactic administration.

The term "physiological disorder associated with β-amyloid peptide" includes diseases related to the inappropriate or undesirable deposition, such as in the brain, liver, kidney, or other organ, of β-amyloid peptide, and as such includes Alzheimer's Disease (including familial Alzheimer's Disease), Down's Syndrome, advanced aging of the brain, hereditary cerebral hemorrhage with amyloidosis of the Dutch-type (HCHWA-D), and the like.

The term "effective amount" as used herein refers to the amount of compound necessary to inhibit physiological effects or disorders associated with β-amyloid peptide, or inhibit β-amyloid peptide production or deposition, or inhibit Alzheimer's Disease, as the case may be.

The term "conservative variant" as used herein refers to amino acid substitutions which alter the primary sequence of the peptide but do not alter its secondary structure or diminish its amyloidogenic properties.

The terms "β-amyloid(1-43)", "β-amyloid(25-35)" and the like refer to amino acids 1 to 43 or amino acids 25-35 of the β-amyloid peptide.
In all such references, the first number listed is the amino-terminus of this peptide sequence. The term "β-amyloid(40-1)" refers to an amino acid compound comprising the amino acids which correspond to amino acids 1 to 40 of β-amyloid peptide, but in the non-natural orientation wherein amino acid 40 is at the amino terminus. This compound is frequently used as a negative control.

The bafilomycins are a series of unusual macrolide antibiotics with a 16-membered lactone ring which have traditionally been isolated from the microorganism Streptomyces griseus. G. Werner, et al., Journal of Antibiotics, 37:100-117 (1984). These antibiotics are effective as specific inhibitors of vacuolar adenosinetriphosphatases (V-type ATPases), showing moderate or no inhibition of other ATPases. S. Dröse, et al., Biochemistry, 32:3902-3906 (1993); and E.J. Bowman, et al., Proceedings of the National Academy of Sciences (USA), 85:7972-7976 (1988).

The most widely characterized bafilomycin is designated as bafilomycin $A_1$ and has the formula.

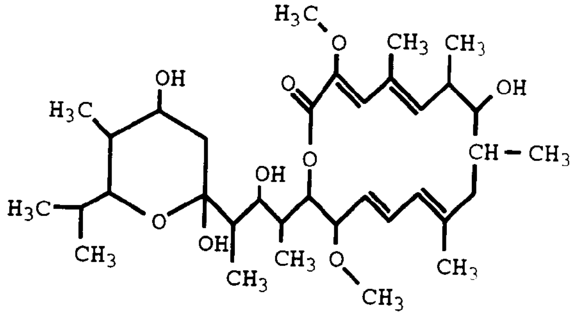

Bafilomycin A1

Other members of the bafilomycin family which are effective inhibitors of vacuolar ATPases are the following compounds.

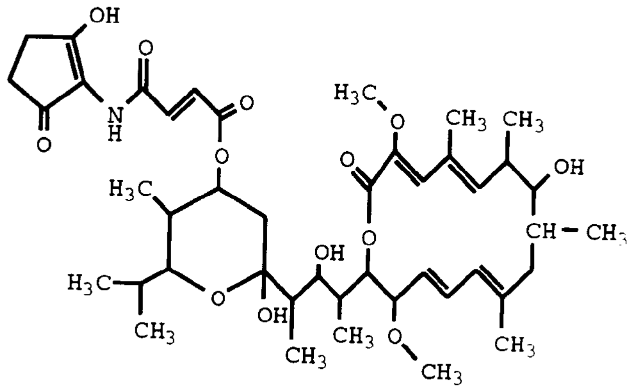

Bafilomycin B1

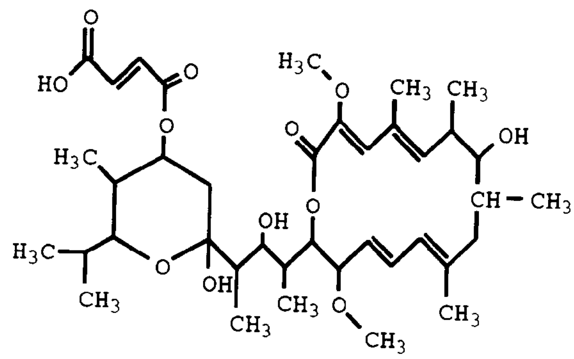

Bafilomycin C1

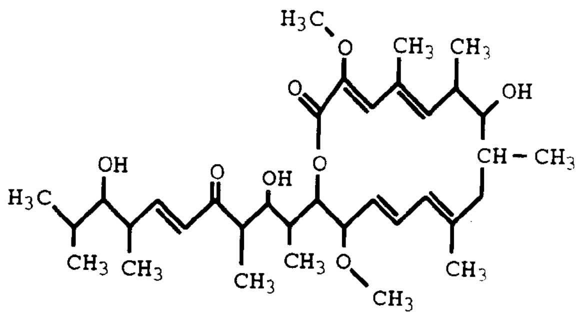

Bafilomycin D

The compound bafilomycin D is a significantly poorer (~40 fold) inhibitor of vacuolar ATPases than other bafilomycins, especially bafilomycin A1. The bafilomycins can be synthesized by well known methodologies using readily accessible microorganisms. See, e.g., U.S. Patent 4,558,139, issued December 10, 1985 to H-P. Hagenmaier, et al., herein incorporated by reference; and E.J. Bowman, et al., Proceedings of the National Academy of Sciences (USA), 85:7972-7976 (1988). In addition, the bafilomycins are available from commercial sources.

Another series of antibiotics useful as inhibitors of vacuolar adenotriphosphatases are the concanamycins, a series having an 18-membered lactone ring and a 6-membered hemiketal ring in which the hydroxy group is glycosylated by 2-deoxy-β-D-rhamnose H. Kinashi, et al., Journal of Antibiotics, 37:1333-1342 (1984). The concanamycins are typified by concanamycin A, a compound having the following structure.

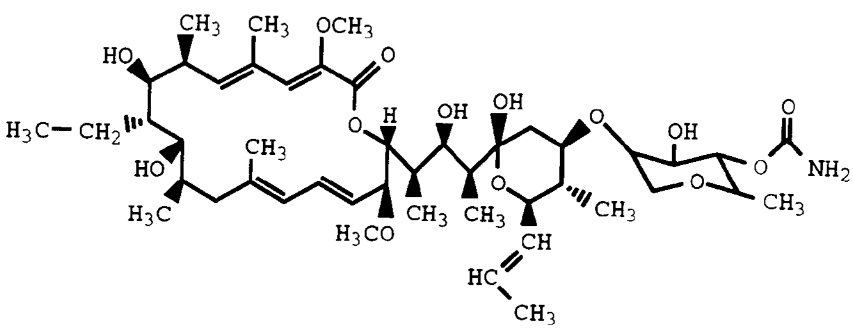

A particularly preferred method of the present invention employs a compound of the formula

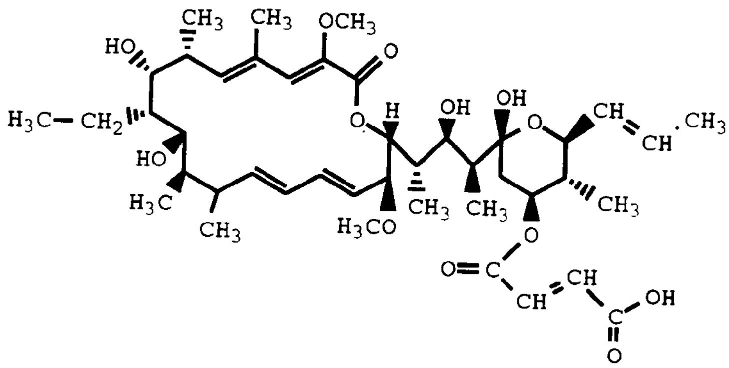

which is hereinafter designated as A87515A. R. Bonjouklian, et al., Abstracts of Papers of the 204th American Chemical Society National Meeting, August 23-28, 1992.

This compound was isolated by growing one liter of Actinomycetes sp. A87515 and then filtering the whole broth through diatomaceous earth. The mycelial cake was then extracted with one liter of methanol. The extract (580 mg) was chromatographed over an LH-20 column (2.5 cm i.d. x 1 m) in methanol. The fractions showing antimicrobial activity against Neurospora crassa using standard disk assays were combined and the solvents were removed in vacuo. The resulting residue (176 mg) was chromatographed over a $C_{18}$ hydrophobic interaction column using a gradient beginning with 70:30 methanol:0.2% acetic acid (pH adjusted to 4.8 with sodium hydroxide) and ending with a 95:5 ratio. The active fractions were concentrated in vacuo and then extracted with ethyl acetate. The solvents were removed in vacuo to yield 17 mg of purified A87515A.

Another preferred method of this invention employs a compound of the formula.

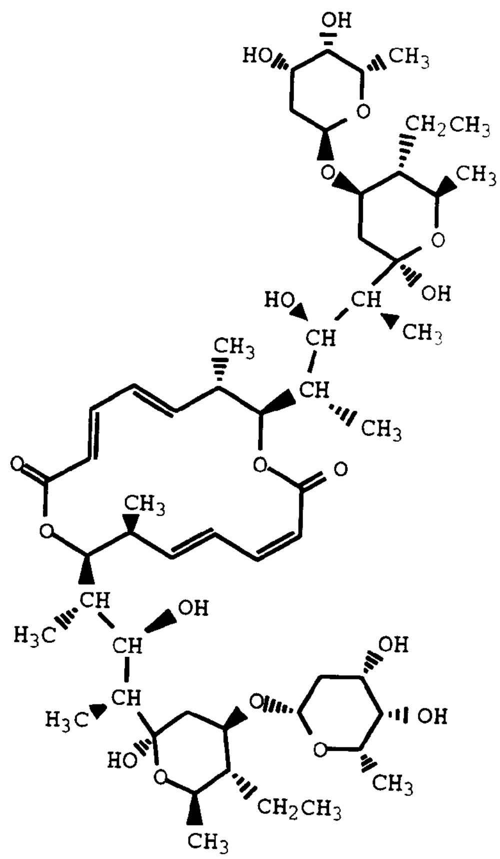

This compound, a macrodiolide with a 16-membered dilactone ring, is usually referred to as elaiophyline or azalomycin B. This compound was first isolated in 1959 from a strain of Streptomyces violaceusniger but the structure was not completely elucidated until 1981. H. Kaiser, et al., Helvetica Chimica Acta, 64:407-424 (1981). Derivatives of this compound which increase its anti-helminthic activity are well known in the literature. See. e.g., U.S. Patent 5,011,827, issued April 30, 1991. The starting material used to produce this compound is known and readily available to the public.

In another embodiment this invention encompasses methods employing the pharmaceutically acceptable salts of

the compounds of Formula I. The term "pharmaceutically acceptable salt" as used herein, refers to salts of the compounds of Formula I which are substantially non-toxic to living organisms. Typical pharmaceutically acceptable salts include those salts prepared by reaction of the compounds of the present invention with a pharmaceutically acceptable organic or inorganic base. Such salts are known as base addition salts.

Base addition salts include those derived from inorganic bases, such as ammonium or alkali or alkaline earth metal hydroxides, carbonates, bicarbonates, and the like. Such bases useful in preparing the salts of this invention thus include sodium hydroxide, potassium hydroxide, ammonium hydroxide, potassium carbonate, sodium carbonate, sodium bicarbonate, potassium bicarbonate, calcium hydroxide, calcium carbonate, and the like. The potassium and sodium salt forms are particularly preferred. Organic bases can also be used, including primary, secondary, and tertiary alkyl amines such as methylamine, triethylamine, and the like.

It should be recognized that the particular counterion forming a part of any salt of this invention is not of a critical nature, so long as the salt as a whole is pharmacologically acceptable and as long as the counterion does not contribute undesired qualities to the salt as a whole.

This invention also encompasses the pharmaceutically acceptable prodrugs of the compounds of Formula I. Prodrugs are especially favored in the treatment or prevention of physiological disorders associated with an amyloidogenic peptide in the central nervous system, due to the necessity of permeation through the blood-brain barrier.

The blood-brain barrier results from the endothelial cells in the capillaries of the brain being joined by continuous, tight intercellular junctions, such that materials must pass through the cells rather than between them in order to move from the blood to the brain. Because of the existence of this barrier, foreign compounds which enter other organs with ease may penetrate the central nervous system slowly or hardly at all. Penetration of this barrier may occur by several methods: lipid soluble substances may passively penetrate into the cells; water, urea and other such small molecules may pass through pores; and specific molecules may move through the barrier by active transport and carrier-mediated means.

A prodrug is a drug which has been chemically modified and may be biologically inactive at its site of action, but which may be degraded or modified by one or more enzymatic or other in vivo processes to the parent bioactive form. This prodrug should have a different pharmacokinetic profile to the parent, enabling easier absorption across the mucosal epithelium, better salt formation or solubility, improved systemic stability (for an increase in plasma half-life, for example). Typical such chemical modifications include:

1) ester or amide derivatives which may be cleaved by esterases or amidases;

2) peptides which may be recognized by specific or nonspecific proteases; or

3) derivatives that accumulate at a site of action through membrane selection of a prodrug form or a modified prodrug form;

or any combination of 1 to 3, supra. Conventional procedures for the selection and preparation of suitable prodrug derivatives are described, for example, in "Design of Prodrugs", (H, Bundgaard, ed., 1985).

Current research in animal experiments has shown that the absorption and permeability of certain drugs may be increased by the preparation of "soft" quarternary salts. See, e.g., D. Horwell, Patent Cooperation Treaty Publication WO 92/04038, published March 19, 1992. The quarternary salt is termed a "soft" quarternary salt since, unlike normal quarternary salts, e.g., $R\text{-}N^{+}(CH_3)_3$, it can release the active drug on hydrolysis.

A preferred embodiment of this invention employs prodrugs described in U.S. Patent 4,900,837, herein incorporated by reference. These prodrugs are generally prepared by the reaction of the compound of Formula I, or an appropriately protected derivative thereof, with isonicotinoyl chloride, with isonicotinic anhydride, or with isonicotinic acid in the presence of a suitable coupling agent such as dicyclohexylcarbodiimide, in an appropriate organic solvent to afford the corresponding isonicotinamide. The isonicotinamide is then quarternized, typically by treatment with methyl iodide in a suitable organic solvent, to afford the quarternary derivative. The quarternary derivative is then reduced by treatment with sodium dithionate or sodium borohydride to afford the desired compound.

In an alternative embodiment of this invention the derivatized compounds of Formula I may be prepared using picolinic acid or its acid chloride or anhydride, or nicotinic acid or its acid chloride or anhydride, in place of isonicotinic acid or its acid chloride or anhydride, respectively, to convert the compounds of Formula I to the corresponding picolinamides and nicotinamides.

Alternatively, a compound of Formula I may be reacted with an activated ester of nicotinic acid, picolinic acid, or isonicotinic acid, e.g. a succinimidyl ester such as

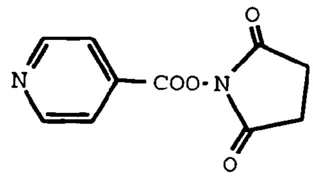

and the procedure described above repeated to afford the identical products. As yet another alternative, the activated ester, e.g. the succinimidyl ester depicted above may be quaternized (e.g. by treatment with methyl iodide) and the quaternized activated ester then reacted with a compound of Formula I. The quaternary compound thus obtained may then be reduced as described above.

These prodrugs have the advantage of being better able to cross the blood-brain barrier due to their increased lipophilicity. Upon entry into the cerebrospinal fluid or the extracellular fluid of the brain, amidases may cleave the isonicotinamide group, leaving the free compound of Formula I.

In alternative embodiment of this invention prodrugs may be employed in which a compound of Formula I is conjugated with a protein which normally crosses the blood-brain barrier by active transport nor carrier-mediated means. This conjugation may be most easily accomplished through a primary amino or hydroxy group on the compound of Formula I by coupling with a reactively available amino, hydroxy, or thiol group on the protein using standard protein coupling techniques and reagents.

Another derivatization of the compounds of Formula I which may be employed as prodrugs in the methods of this invention employs the phosphonate moieties of U.S. Patent 5,177,064, issued January 5, 1993, which is herein incorporated by reference. These derivatives are adapted for targeted drug delivery, especially to the brain.

The ability of the prodrugs of this invention to pass through the blood-brain barrier may be approximated by numerous means known to those skilled in the art. One such widely used assay is partitioning between octanol and water, those compounds of sufficient hydrophobicity to pass through the blood-brain barrier being more soluble in octanol. Another method of predicting passage through the this barrier with a great deal of accuracy is by use of the in vitro blood-brain barrier model described in U.S. Patent 5,260,210, issued November 9, 1993, which is herein incorporated by reference. This model provides for the rapid assessment of the ability of a particular prodrug to permeate the blood-brain barrier.

β-amyloid peptide naturally occurs as a series of peptides which are 39 to 43 amino acids long, with the shorter, more soluble forms being present in cerebrovascular deposits and the longer forms being found primarily in senile plaques. F. Prelli, et al., Journal of Neurochemistry, 51:648-651 (1988). The primary structure of the 43 amino acid long peptide.

Even though this form of β-amyloid peptide can be found in nature, there are also found various amino-terminal deletions and carboxy-terminal deletions which result in a considerable mixture of different forms of β-amyloid peptide which differ in length. These various forms are referred to collectively as β-amyloid peptide.

While the peptide described supra and the deletants of this peptide are referred to as β-amyloid peptide throughout this document, in the body of literature concerning this field, this peptide is alternatively referred to as β-amyloid protein, amyloid β peptide, amyloid βA4, β protein, amyloid A4, β-peptide, and other such names.

## β-Amyloid Peptide Production Inhibition (Cellular Assay)

Two cell lines (human kidney cell line 293 and Chinese hamster ovary cell line CHO) were stably transfected with the gene for APP751 containing the double mutation $Lys_{651}$-$Met_{652}$ to $Asn_{651}$-$Leu_{652}$ (APP-751 numbering) commonly called the Swedish mutation using the method described in Citron, et al., Nature (London), 360:672-674 (1992). The transfected cell lines were designated as 293 751 SWE and CHO 751 SWE, and were plated in Corning 96 well plates at 2.5 x $10^4$ or 1 x $10^4$ cells per well respectively in Dulbecco's minimal essential media (DMEM) plus 10% fetal bovine serum. Following overnight incubation at 37°C in an incubator equilibrated with 10% carbon dioxide ($CO_2$) and humidified air, the media were removed and replaced with 200 µl per well of media containing a macrolide. After a two hour pretreatment period, the media were again removed and replaced with fresh media containing the test compound and the cells were incubated for an additional two hours.

Macrolide stocks were prepared in dimethylsulfoxide such that at the final concentration used in the treatment, the concentration of dimethylsulfoxide did not exceed 0.5%. After treatment, plates were centrifuged at 1200 x g for five minutes at room temperature to pellet cellular debris from the conditioned media. From each well, 100 µl of conditioned media were transferred into an ELISA plate precoated with antibody 266 against βamyloid peptide(13-28) (Seubert et al., supra.) and stored at 4°C overnight. An ELISA assay employing labelled antibody 6C6 (against β-amyloid peptide-1-16) was run the next day to measure the amount of β-amyloid peptide produced.

Cytotoxic effects of the compounds were measured by a modification of the method of Hansen, et al., Journal of Immununological Methods, 119:203-210 (1989). To the cells remaining in the tissue culture plate, was added 25 µl of

a 3-(4,5-dimethylthiazol-2-yl)2,5-diphenyltetrazolium bromide (MTT) stock solution (5 mg/ml) to a final concentration of 1 mg/ml. Cells were incubated at 37°C for one hour, and cellular activity was stopped by the addition of an equal volume of MTT lysis buffer (20% w/v sodium dodecylsulfate in 50% DMF, pH 4.7). Complete extraction was achieved by overnight shaking at room temperature. The difference in the $OD_{562nm}$ and the $OD_{650nm}$ was measured in a $UV_{max}$ microplate reader as an indicator of the cellular viability.

The results of the β-amyloid peptide ELISA were fit to a standard curve and expressed as ng/ml β-amyloid peptide. In order to normalize for cytotoxicity, these β-amyloid peptide results were divided by the MTT results and expressed as a percentage of the results from a drug-free control.

Table 1

| β-Amyloid Peptide Production Inhibition Activity in Vitro | | | |
|---|---|---|---|
| Compound | Concentration (μM) | Percent Inhibition | Percent Viability |
| Bafilomycin $A_1$ | 0.3 | 90 ± 2 | 112 |
| | 0.3 | 91 ± 1 | 99 |
| | 0.3 | 92 ± 1 | 102 |
| Bafilomycin $B_1$ | 1 μg/ml | 87 ± 1 | |
| Bafilomycin $C_1$ | 1 μg/ml | 90 ± 2 | |
| Bafilomycin D | 1 μg/ml | 4 ± 3 | |
| Concanamycin A | 1.2 | 74 ± 4 | 97 |
| Elaiophylin | 9.8 | 131 ± 1 | 63 |
| | 0.6 | 98 ± 2 | 96 |
| | 1.2 | 100 ± 1 | 95 |
| | 2.4 | 100 ± 2 | 69 |
| | 4.9 | 100 ± 1 | 68 |
| | 9.8 | 100 ± 1 | 65 |
| A87515A | 12.6 | 108 ± 4 | 95 |
| | 0.8 | 88 ± 2 | 111 |
| | 1.6 | 89 ± 1 | 103 |
| | 3.2 | 89 ± 1 | 109 |
| | 6.3 | 86 ± 2 | 114 |
| | 12.6 | 86 ± 0 | 113 |

In some experiments dealing with other forms of the amyloid precursor protein, such as the "wild-type" form of the protein, in the 293 cell line, the addition of the vacuolar adenosinetriphosphatase inhibitors described supra do not have the same effects as those 293 cells transfected with the Swedish mutation.

In order to better understand this phenomenon, human fetal primary cortical cultures, which secrete β-amyloid peptide as a result of endogenous amyloid precursor protein processing, were treated with 1 μM bafilomycin. Twenty four hour-conditioned medium was collected and evaluated using enzyme-linked immunosorbent assay (ELISA). Greater than 75% inhibition of β-amyloid peptide production was observed in these cultures. At these levels no cytotoxicity was observed.

Like the 293 cells expressing the Swedish mutation form of amyloid precursor protein, β-amyloid peptide production from the human fetal primary cultures expressing genomic wild-type amyloid precursor protein thus appears to be susceptible to the vacuolar adenosinetriphosphatase inhibitors.

In order to better study the effects of these inhibitors of vacuolar ATPases on whole animals, some of the above compounds were administered in vivo to guinea pigs by an intraperitoneal route at 1 mg/kg/day. The individual compound being tested was administered in four 0.25 mg/kg doses separated by one hour. One hour after the last of the four injections, the amount of β-amyloid peptide in the cerebrospinal fluid, using the aforementioned ELISA assay, was measured and compared to control animals in which no such compound was administered.

In one such experiment with the compound A87515A significant lowering of the amount of β-amyloid peptide was observed using this assay.

The compounds of Formula I are usually administered in the form of pharmaceutical compositions. These compounds can be administered by a variety of routes including oral, rectal, transdermal, subcutaneous, intravenous, intramuscular, and intranasal. These compounds are effective as both injectable and oral compositions. Such compositions are prepared in a manner well known in the pharmaceutical art and comprise at least one active compound.

The present invention also includes pharmaceutical compositions which contain, as the active ingredient, the compounds of Formula I associated with pharmaceutically acceptable carriers. In making the compositions of the present invention the active ingredient is usually mixed with an excipient, diluted by an excipient or enclosed within such a carrier which can be in the form of a capsule, sachet, paper or other container. When the excipient serves as a diluent, it can be a solid, semi-solid, or liquid material, which acts as a vehicle, carrier or medium for the active ingredient. Thus, the compositions can be in the form of tablets, pills, powders, lozenges, sachets, cachets, elixirs, suspensions, emulsions, solutions, syrups, aerosols (as a solid or in a liquid medium), ointments containing for example up to 10% by weight of the active compound, soft and hard gelatin capsules, suppositories, sterile injectable solutions, and sterile packaged powders.

In preparing a formulation, it may be necessary to mill the active compound to provide the appropriate particle size prior to combining with the other ingredients. If the active compound is substantially insoluble, it ordinarily is milled to a particle size of less than 200 mesh. If the active compound is substantially water soluble, the particle size is normally adjusted by milling to provide a substantially uniform distribution in the formulation, e.g. about 40 mesh.

Some examples of suitable excipients include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water, syrup, and methyl cellulose. The formulations can additionally include: lubricating agents such as talc, magnesium stearate, and mineral oil; wetting agents; emulsifying and suspending agents; preserving agents such as methyl- and propylhydroxybenzoates; sweetening agents; and flavoring agents. The compositions of the invention can be formulated so as to provide quick, sustained or delayed release of the active ingredient after administration to the patient by employing procedures known in the art.

The compositions are preferably formulated in a unit dosage form, each dosage containing from about 5 to about 100 mg, more usually about 10 to about 30 mg, of the active ingredient. The term "unit dosage form" refers to physically discrete units suitable as unitary dosages for human subjects and other mammals, each unit containing a predetermined quantity of active material calculated to produce the desired therapeutic effect, in association with a suitable pharmaceutical excipient.

The active compound is effective over a wide dosage range. For example, dosages per day normally fall within the range of about 0.05 to about 30 mg/kg of body weight. In the treatment of adult humans, the range of about 0.1 to about 15 mg/kg/day, in single or divided dose, is especially preferred. However, it will be understood that the amount of the compound actually administered will be determined by a physician, in the light of the relevant circumstances, including the condition to be treated, the chosen route of administration, the actual compound administered, the age, weight, and response of the individual patient, and the severity of the patient's symptoms, and therefore the above dosage ranges are not intended to limit the scope of the invention in any way. In some instances dosage levels below the lower limit of the aforesaid range may be more than adequate, while in other cases still larger doses may be employed without causing any harmful side effect, provided that such larger doses are first divided into several smaller doses for administration throughout the day.

For preparing solid compositions such as tablets the principal active ingredient is mixed with a pharmaceutical excipient to form a solid preformulation composition containing a homogeneous mixture of a compound of the present invention. When referring to these preformulation compositions as homogeneous, it is meant that the active ingredient is dipsersed evenly throughout the composition so that the composition may be readily subdivided into equally effective unit dosage forms such as tablets, pills and capsules. This solid preformulation is then subdivided into unit dosage forms of the type described above containing from 0.1 to about 500 mg of the active ingredient of the present invention.

The tablets or pills of the present invention may be coated or otherwise compounded to provide a dosage form affording the advantage of prolonged action. For example, the tablet or pill can comprise an inner dosage and an outer dosage component, the latter being in the form of an envelope over the former. The two components can be separated by enteric layer which serves to resist disintegration in the stomach and permit the inner component to pass intact into the duodenum or to be delayed in release. A variety of materials can be used for such enteric layers or coatings, such materials including a number of polymeric acids and mixtures of polymeric acids with such materials as shellac, cetyl alcohol, and cellulose acetate.

The liquid forms in which the novel compositions of the present invention may be incorporated for administration orally or by injection include aqueous solutions, suitably flavored syrups, aqueous or oil suspensions, and flavored emulsions with edible oils such as cottonseed oil, sesame oil, coconut oil, or peanut oil, as well as elixirs and similar pharmaceutical vehicles.

Compositions for inhalation or insufflation include solutions and suspensions in pharmaceutically acceptable, aqueous or organic solvents, or mixtures thereof, and powders. The liquid or solid compositions may contain suitable pharmaceutically acceptable excipients as described supra. Preferably the compositions are administered by the oral or nasal respiratory route for local or systemic effect. Compositions in preferably pharmaceutically acceptable solvents may be nebulized by use of inert gases. Nebulized solutions may be breathed directly from the nebulizing device or the nebulizing device may be attached to a face mask, tent, or intermittent positive pressure breathing machine. Solution,

suspension, or powder compositions may be administered, preferably orally or nasally, from devices which deliver the formulation in an appropriate manner.

These compounds may also be administered in the form of a lipid emulsion or liposomal prepartion. In a liposome drug deliver system the medicament is entrapped during liposome formation and then administered to the patient to be treated. The medicament may be soluble in water,or in a non-polar solvent.

Generally, such liposomes may be formed by a variety of techniques that start with "dry" lipids that are introduced into an aqueous phase. Once the lipid is hydrated, liposomes form spontaneously. Techniques have been developed to control the number of lamellae in the liposomes and to produce a defined particle size. Pharmaceutical formulations employing liposomes or lipid emulsions have been described in multiple publications. See, e.g., United States Patents 4,053,585, 4,261,975, 4,522,803, 4,588,578, 5,316,771, 4,752,425, 5,270,053, 5,171,755, 5,234,634, 3,993,754, and 4,145,410.

An alternative technique is taught in United States Patent 5,277, 914, issued January 11, 1994, which is herein incorporated by reference. In such preparations a liposome-forming lipid preparation is dissolved in an aprotic solvent such as dimethylsulfoxide, optionally containing a lipid-solubilizing amount of a lower alkanol, and then mixed with the intended drug. The resulting oslution is then injected through a suitably-sized aperture into a stirred or mixed aqueous solution of appropriate ionic strength and drug composition.

Liposomes (phospholipid vesicles) exhibit a wide variety of characteristics, depending upon their size, composition, and charge. For example, liposomes having a small percentage of unsaturated lipids tend to be slightly more permeable, while liposomes incorporating cholesterol or other sterols tend to be more rigid and less permeable. Liposomes may be positive, negative, or neutral in charge, depending on the hydrophilic group. For example, chloine-based lipids impart a positive charge, phosphate- and sulfate-based lipids contribute a negative charge, and glycerol-based lipids and sterols are generally neutral.

The following examples illustrate the pharmaceutical compositions of the present invention.

## Formulation Example 1

Hard gelatin capsules containing the following ingredients are prepared:

| Ingredient | Quantity (mg/capsule) |
|---|---|
| Active Ingredient | 30.0 |
| Starch | 305.0 |
| Magnesium stearate | 5.0 |

The above ingredients are mixed and filled into hard gelatin capsules in 340 mg quantities.

## Formulation Example 2

A tablet formula is prepared using the ingredients below:

| Ingredient | Quantity (mg/tablet) |
|---|---|
| Active Ingredient | 25.0 |
| Cellulose, microcrystalline | 200.0 |
| Colloidal silicon dioxide | 10.0 |
| Stearic acid | 5.0 |

The components are blended and compressed to form tablets, each weighing 240 mg.

## Formulation Example 3

A dry powder inhaler formulation is prepared containing the following components:

| Ingredient | Weight % |
|---|---|
| Active Ingredient | 5 |
| Lactose | 95 |

The active mixture is mixed with the lactose and the mixture is added to a dry powder inhaling appliance.

Formulation Example 4

Tablets, each containing 30 mg of active ingredient, are prepared as follows:

| Ingredient | Quantity (mg/tablet) |
|---|---|
| Active Ingredient | 30.0 mg |
| Starch | 45.0 mg |
| Microcrystalline cellulose | 35.0 mg |
| Polyvinylpyrrolidone (as 10% solution in water) | 4.0 mg |
| Sodium carboxymethyl starch | 4.5 mg |
| Magnesium stearate | 0.5 mg |
| Talc | 1.0 mg |
| Total | 120 mg |

The active ingredient, starch and cellulose are passed through a No. 20 mesh U.S. sieve and mixed thoroughly. The solution of polyvinylpyrrolidone is mixed with the resultant powders, which are then passed through a 16 mesh U.S. sieve. The granules so produced are dried at 50-60°C and passed through a 16 mesh U.S. sieve. The sodium carboxymethyl starch, magnesium stearate, and talc, previously passed through a No. 30 mesh U.S. sieve, are then added to the granules which, after mixing, are compressed on a tablet machine to yield tablets each weighing 150 mg.

Formulation Example 5

Capsules, each containing 40 mg of medicament are made as follows:

| Ingredient | Quantity (mg/capsule) |
|---|---|
| Active Ingredient | 40.0 mg |
| Starch | 109.0 mg |
| Magnesium stearate | 1.0 mg |
| Total | 150.0 mg |

The active ingredient, cellulose, starch, and magnesium stearate are blended, passed through a No. 20 mesh U.S. sieve, and filled into hard gelatin capsules in 150 mg quantities.

Formulation Example 6

Suppositories, each containing 25 mg of active ingredient are made as follows:

| Ingredient | Amount |
|---|---|
| Active Ingredient | 25 mg |
| Saturated fatty acid glycerides to | 2,000 mg |

The active ingredient is passed through a No. 60 mesh U.S. sieve and suspended in the saturated fatty acid glycerides previously melted using the minimum heat necessary. The mixture is then poured into a suppository mold of nominal 2.0 g capacity and allowed to cool.

Formulation Example 7

Suspensions, each containing 50 mg of medicament per 5.0 ml dose are made as follows:

| Ingredient | Amount |
|---|---|
| Active Ingredient | 50.0 mg |
| Xanthan gum | 4.0 mg |
| Sodium carboxymethyl cellulose (11%) | |
| Microcrystalline cellulose (89%) | 50.0 mg |
| Sucrose | 1.75 g |
| Sodium benzoate | 10.0 mg |
| Flavor and Color | q.v. |
| Purified water to | 5.0 ml |

The medicament, sucrose and xanthan gum are blended, passed through a No. 10 mesh U.S. sieve, and then mixed with a previously made solution of the microcrystalline cellulose and sodium carboxymethyl cellulose in water. The sodium benzoate, flavor, and color are diluted with some of the water and added with stirring. Sufficient water is then added to produce the required volume.

Formulation Example 8

Capsules, each containing 15 mg of medicament, are made as follows:

| Ingredient | Quantity (mg/capsule) |
|---|---|
| Active Ingredient | 15.0 mg |
| Starch | 407.0 mg |
| Magnesium stearate | 3.0 mg |
| Total | 425.0 mg |

The active ingredient, cellulose, starch, and magnesium stearate are blended, passed through a No. 20 mesh U.S sieve, and filled into hard gelatin capsules in 560 mg quantities.

Formulation Example 9

An intravenous formulation may be prepared as follows:

| Ingredient | Quantity |
|---|---|
| Active Ingredient | 250.0 mg |
| Isotonic saline | 1000 ml |

Formulation Example 10

A topical formulation may be prepared as follows:

| Ingredient | Quantity |
|---|---|
| Active Ingredient | 1-10 g |
| Emulsifying Wax | 30 g |
| Liquid Paraffin | 20 g |
| White Soft Paraffin | to 100 g |

The white soft paraffin is heated until molten. The liquid praffin and emulsifying wax are incorporated and stirred until dissolved. The active ingredient is added and stirring is continued until dispersed. The mixture is then cooled until solid.

Formulation Example 11

Sublingual or buccal tablets, each containing 10 mg of active ingredient, may be prepared as follows:

| Ingredient | Quantity Per Tablet |
|---|---|
| Active Ingredient | 10.0 mg |
| Glycerol | 210.5 mg |
| Water | 143.0 mg |
| Sodium Citrate | 4.5 mg |
| Polivinyl Alcohol | 26.5 mg |
| Polyvinylpyrrolidone | 15.5 mg |
| Total | 410.0 mg |

The glycerol, water, sodium citrate, polyvinyl alcohol, and polyvinylpyrrolidone are admixed together by continuous stirring and maintaining the temperature at about 90°C. When the polymers have gone into solution, the solution is cooled to about 50-55°C and the medicament is slowly admixed. The homogenous mixture is poured into forms made of an inert material to produce a drug-containing diffusion matrix having a thickness of about 2-4 mm. This diffusion matrix is then cut to form individual tablets having the appropriate size.

Another preferred formulation employed in the methods of the present invention employs transdermal delivery devices ("patches"). Such transdermal patches may be used to provide continuous or discontinuous infusion of the compounds of the present invention in controlled amounts. The construction and use of transdermal patches for the delivery of pharmaceutical agents is well known in the art. See. e.g., U.S. Patent 5,023,252, issued June 11, 1991, herein incorporated by reference. Such patches may be constructed for continuous, pulsatile, or on demand delivery of pharmaceutical agents.

Frequently, it will be desirable or necessary to introduce the pharmaceutical composition to the brain, either directly or indirectly. Direct techniques usually involve placement of a drug delivery catheter into the host's ventricular system to bypass the blood-brain barrier. One such implantable delivery system, used for the transport of biological factors to specific anatomical regions of the body, is described in U.S. Patent 5,011,472, issued April 30, 1991, which is herein incorporated by reference.

Indirect techniques, which are generally preferred, usually involve formulating the compositions to provide for drug latentiation by the conversion of hydrophilic drugs into lipid-soluble drugs or prodrugs. Latentiation is generally achieved through blocking of the hydroxy, carbonyl, sulfate, and primary amine groups present on the drug to render the drug more lipid soluble and amenable to transportation across the blood-brain barrier. Alternatively, the delivery of hydrophilic drugs may be enhanced by intra-arterial infusion of hypertonic solutions which can transiently open the blood-brain barrier.

**Claims**

1. The use of a compound having activity as an inhibitor of a vacuolar adenosinetriphosphatase, or a pharmaceutically acceptable salt or prodrug thereof, in the preparation of a medicament for treating or preventing a physiological disorder associated with β-amyloid peptide.

2. A use as claimed in **Claim 1** employing a compound of the formula:

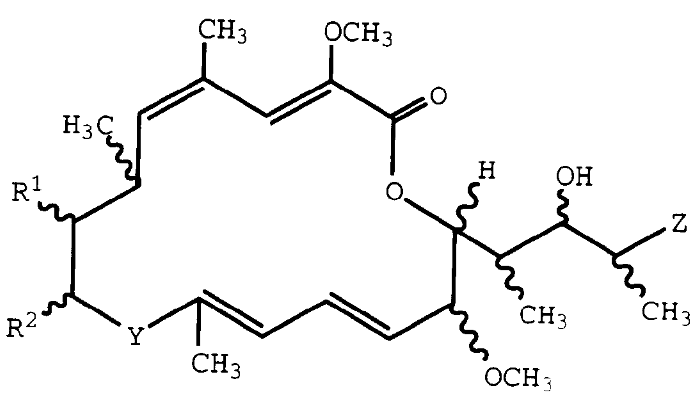

;

wherein:

Y is

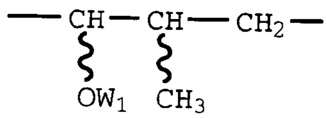 ,

or $-CH_2-$ ;

Z is

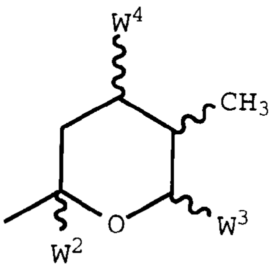 , or 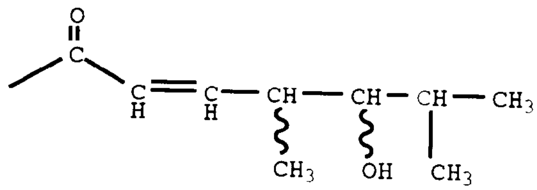 ;

$R^1$ is hydroxy or $C_2$-$C_6$ alkanoyloxy;

$R^2$ is $C_1$-$C_6$ alkyl;

$W^1$ is hydrogen or $C_2$-$C_6$ alkanoyl;

$W^2$ is hydrogen, hydroxy, or $C_1$-$C_6$ alkoxy;

$W^3$ is $C_1$-$C_6$ alkyl, or $C_2$-$C_8$ alkenyl;

$W^4$ is hydroxy, $C_1$-$C_6$ alkoxy, $C_2$-$C_6$ alkanoyloxy,

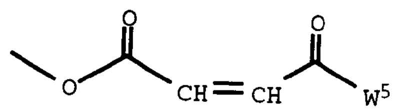 ,

=O , or

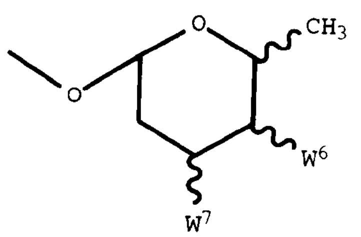 ;

$W^5$ is hydroxy, $C_1$-$C_6$ alkoxy, amino,

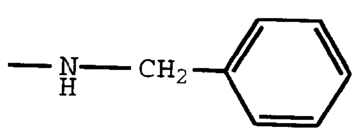 , or 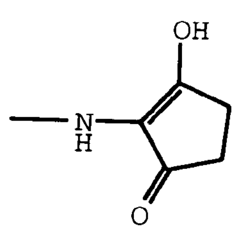 ;

$W^6$ is hydroxy or $C_2$-$C_6$ alkanoyloxy;

$W^7$ is hydroxy or $C_2$-$C_6$ alkanoyloxy;

or a pharmaceutically acceptable salt, solvate, or prodrug thereof.

**3.** The use of a compound selected from the formulae:

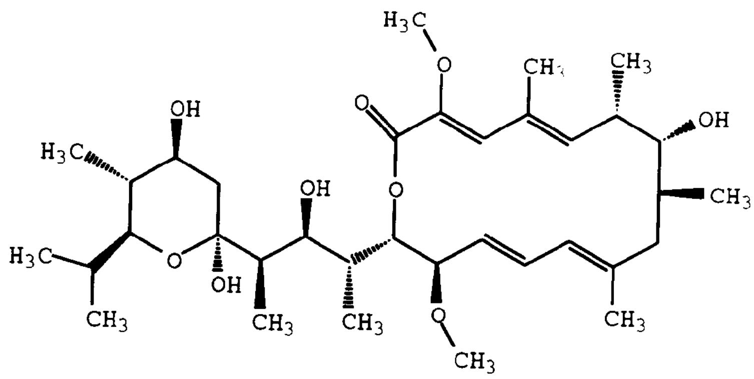

;

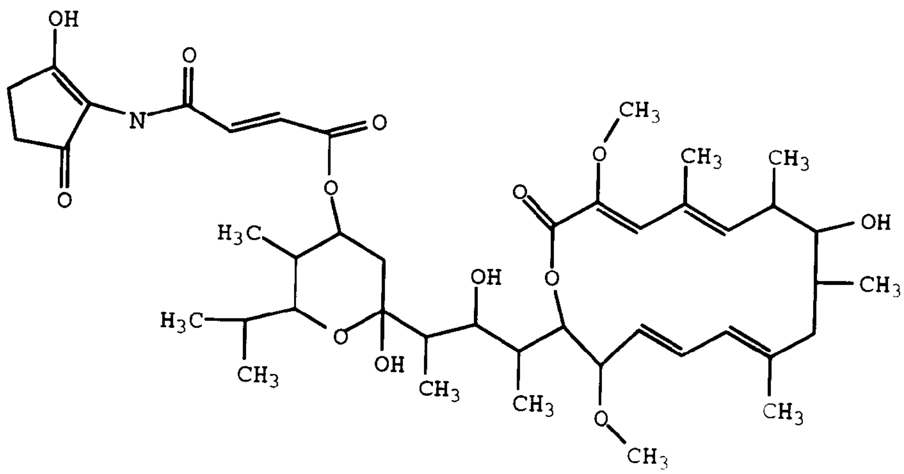

;

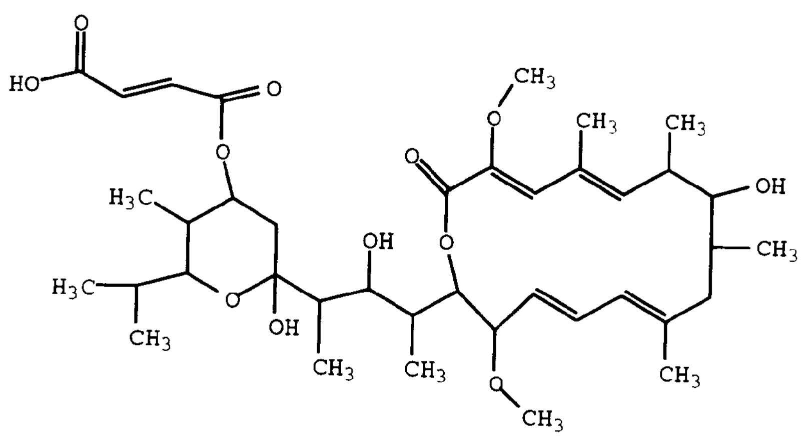

;

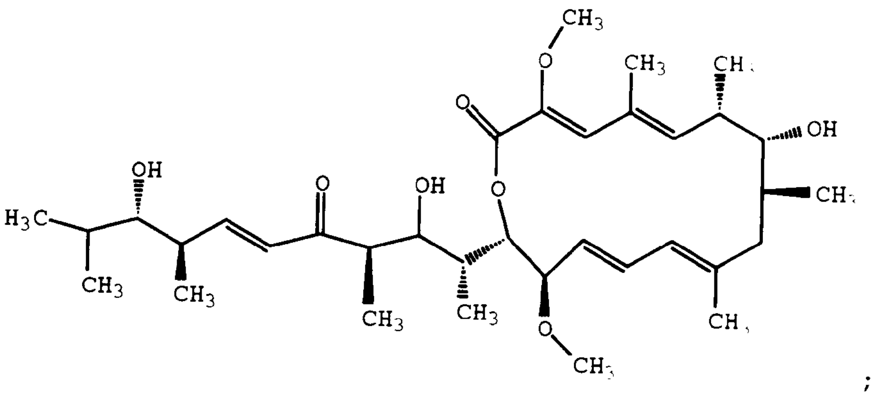

;

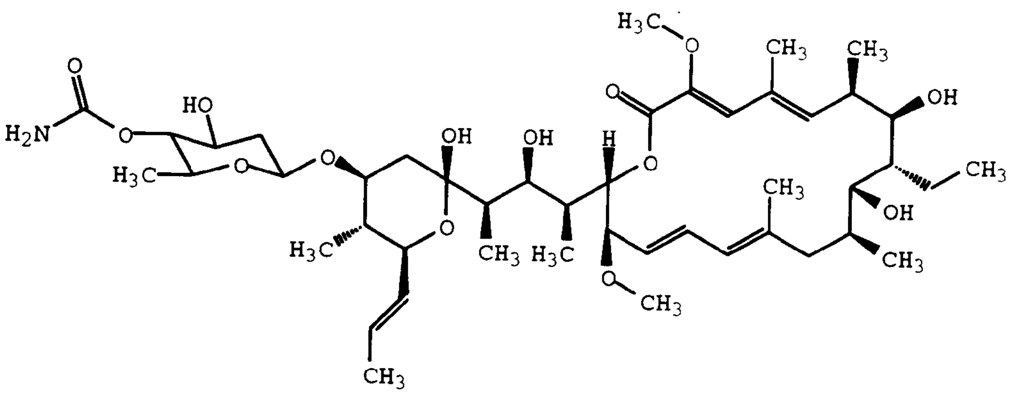

;

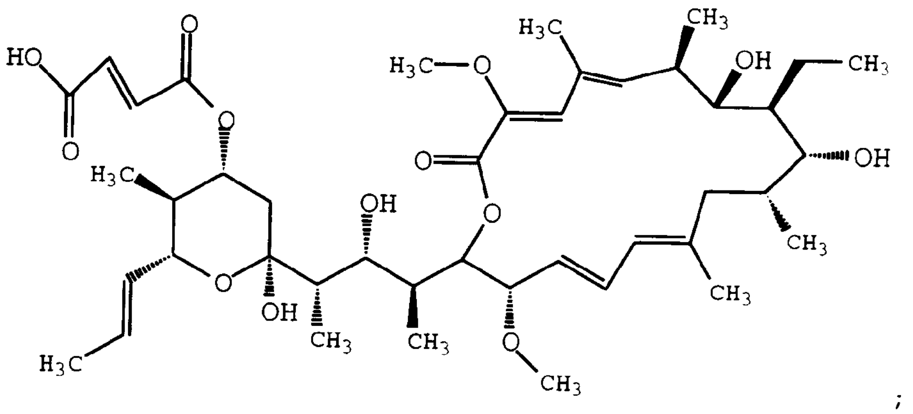

;

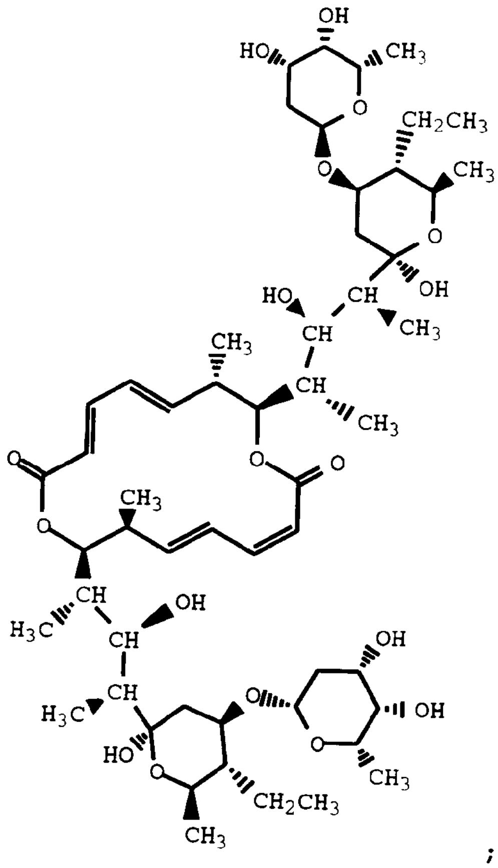

;

or a pharmaceutically acceptable salt, solvate, or prodrug thereof, in the preparation of a medicament for treating or preventing a physiological disorder associated with β-amyloid peptide.